# EUROPEAN PATENT SPECIFICATION

(11) **EP 1 638 458 B1**
(45) Date of publication and mention of the grant of the patent: **02.11.2011**
(21) Application number: 04755539.6
(22) Date of filing: 18.06.2004
(51) Int. Cl.: A61B 5/0484

(54) **DEVICE AND METHOD FOR AN AUTOMATED E.E.G. SYSTEM FOR AUDITORY EVOKED RESPONSES**
VORRICHTUNG UND VERFAHREN FÜR EIN AUTOMATISCHES EEG-SYSTEM FÜR AUDITORISCH EVOZIERTE ANTWORTEN
DISPOSITIF ET PROCEDE POUR SYSTEME AUTOMATISE D'EEG PERMETTANT D'OBTENIR DES REPONSES AUDITIVES

(30) Priority: 19.06.2003 US 479684 P; 29.03.2004 US 557230 P
(43) Date of publication of application: 29.03.2006
(73) Proprietor: Neuronetrix Solutions, LLC, Louisville KY 40204 (US)
(72) Inventor: FADEM, Kalford, C., Louisville, KY 40206 (US)
(74) Representative: Loisel, Bertrand
(86) International application number: PCT/US2004/019418
(87) International publication number: WO 2004/112604

(56) References cited:
- EP-A- 1 238 629
- WO-A-00/66209
- US-A- 5 755 230
- US-A- 5 954 667

## Description

### Field of the Invention

The present invention relates generally to a method and apparatus for capturing electroencephalogram (EEG) signals. More particularly, the present invention provides a method and describes a system for the purpose of diagnosing dyslexia, and similar neurological conditions such as autism, schizophrenia, etc., by capturing brain waves produced while processing a preprogrammed auditory stimulus.

### Background of the Invention

Dyslexia is an inborn condition characterized by abnormal brain physiology or "defective wiring". Detailed studies of the brains of known dyslexics show a marked difference from normal brains. This physical difference has been detected with a variety of brain imaging modalities including: MRI, CT, and PET. The pathophysiology is characterized by a disruption in left hemisphere posterior reading systems, primarily in left temporo-parieto-occipital brain regions, with a relative increase in brain activation in frontal regions (Shaywitz, et al, "Dyslexia Specific Reading Disability", Pediatrics in Review, Vol. 24, No. 5, May 2003).

Dyslexia effects about 10-15% of the population to varying degrees and manifests as difficulty reading, inability to concentrate, and various other learning disabilities. Surprisingly, dyslexia is unrelated to low I.Q. and is common in many successful and motivated people. Examples of recognized dyslexics include Einstein, Edison, Ford, Patton, da Vinci, Rockefeller, Churchill, Disney, and others (from www.dyslexia.com). Dyslexia is not a chemical imbalance or behavioral disorder like Attention Deficit Hyperactivity Disorder (ADHD) and can't be treated with drugs such as Ritalin as used in some ADHD children.

Typically, dyslexia is not diagnosed until between ages 5 and 8, usually after the child has fallen two grade levels behind in reading. By this time, much of the permanent damage to the child has already been done. Parents of dyslexic children are often forced to endure the difficulties of raising a child who has been labeled as "slow", "disruptive", or a "problem-child". Sometimes these dyslexic children are misdiagnosed as ADHD. These children may be put on drug therapy in hopes of controlling disruptive behaviors. While this may have some mitigating impact on the school system, this kind of therapy will have no beneficial direct effect on the dyslexia itself. The longer-term negative effects include illiteracy, anti-social behavior, and low income.

Recent evidence has shown that dyslexic brains can be remodeled or retrained to overcome the wiring defect (Simos, et al., "Dyslexia-specific brain activation profile becomes normal following successful remedial training", Neurology 2002; 58:1203-1213). Many experts believe that intervention will be most effective early in a child's life ("A New Era: Revitalizing Special Education for Children and Their Families", President's Commission on Excellence in Special Education, July 1, 2002). Early detection is the key mitigating the lifelong effects of dyslexia.

Most current dyslexia screening tests measure obscure, anecdotal, action- response behaviors. Some tests include posturography which measures balance strategies, bead threading which measures sequential memory, rhyming games which measure phonological awareness, and others. These tests can only be given to a child who is old enough to perform reading, puzzle solving, or other high-level assessment skills. None of these tests directly detect the underlying physical brain wiring defect. Poor performance on these tests could be attributed to causes other than dyslexia.

In 1929, the German psychiatrist, Hans Berger, announced to the world that: "it was possible to record the feeble electric currents generated on the brain, without opening the skull, and to depict them graphically onto a strip of paper... that this activity changed according to the functional status of the brain, such as in sleep, anesthesia, hypoxia (lack of oxygen) and in certain nervous diseases, such as in epilepsy." (Berger, H., "Uber das elektrenkephalogramm des menschen", Archiv fur Psychiatrie und NervenkranEheiten, 1929, 87:527-580). Berger named this new form of recording as the electroencephalogram (EEG).

Electroencephalograms or EEG's are voltage potentials measured on the scalp produced during brain activity where the magnitude of the voltage differentials is plotted versus time. An EEG system is composed of several discrete system components. The first component is a conductive electrode that is placed on the scalp generally in close proximity to an inactive part of the brain. This inactive or "reference" electrode is used as a reference for other "active" electrodes placed on the scalp in close proximity to processing areas of the brain. The electrodes are electrically connected to voltage amplifiers, bandpass filters, and various other electronic components generally used in processing electronic signals. In most current systems, the analog voltage signal is passed through an analog to digital converter where the signal is sampled at a user-controlled rate and converted to digital data. The digital data may then be stored on digital media for later processing.

The most important requirements for clinical application of EEG's were described in 1947 in U.S. Patent No. 2,426,958 by Ulett. The electrode and placement technique associated therewith should be such that the electrode produces no artifacts; is easy to apply, keep on, and remove; and, it is relatively cheap in production and painless in its application and use.

EEG measurements from auditory evoked responses (AER) detect voltage potentials from the brain as the brain attempts to discriminate a sound. EEG's from dyslexic children show abnormally high peak voltages and signal latencies. These characteristics correlate to higher than normal energy requirements to process sounds and slower discrimination and sound-to-symbol mapping, the outward manifestations of which will primarily be difficulty in reading and writing.

A body of research has been performed by Drs. Dennis and Victoria Molfese to develop and scientifically validate the use of AER's to diagnose dyslexia in infants so that earlier and more effective intervention is possible. (Molfese, D., "Predicting Dyslexia at 8 Years of Age Using Neonatal Brain Responses", Brain and Language 72, 238-245 (2000); Molfese, et al, "Newborn and Preschool Predictors of Second-Grade Reading Scores", Journal of Learning Disabilities, No. 6, November/December 2001, pp 545-554; and Molfese, et al, "The Use of Brain Electrophysiology Techniques to Study Language: A Basic Guide for the Beginning Consumer of Electrophysiology Information", Learning Disability Quarterly, Volume 24, Summer 2001, pp. 177-188). In particular, this research has identified optimum positions on the subject's scalp for picking up these characteristic electrical potentials and for identifying an AER characteristic of dyslexia.

While this research into AER diagnosis of dyslexia has been of scientific interest, diagnosis of dyslexia in infants is not common in a clinical setting. By contrast, hearing deficits affect only about 1 in 500 newborns whereas dyslexia affects 50 times as many children, yet the Universal Newborn Hearing Screening (UNHS) test is mandated in 38 states. Therefore, approximately 69% of the 4,000,000 children born in the U.S. each year have this UNHS test performed soon after birth. The UNHS test uses a similar EEG technology to measure brainwaves from the brain stem or "unconscious" part of the brain as a result of an auditory stimulus. Documents US-5954 667 and EP-1 238 629, accordingly to the respective preambles of claims 1 and 23, disclose a device for derivation of acoustically evoked hain potentials for testing hearing and for evaluating of acousticaly evoked hain potentials hearing and for evaluating various causes of hearing damage.

At least part of this disparity in clinical use is believed to be the testing equipment required for dyslexia AER diagnosis. The UNHS test only verifies the connection between the ear and the brain stem. As such, a relatively simple diagnostic analysis is required to detect the presence of brain stem response to a sound. The dyslexia AER diagnosis requires a more subtle comparison of characteristic waveforms for optimum auditory processing in the normal population compared to a sub-optimum auditory processing in the dyslexic population. Such sophisticated processing makes this analysis unpractical for the relatively untrained staff in a neonatal care unit.

Moreover, unlike UNHS testing wherein measurements from a single active electrode are used, dyslexia AER testing requires a time consuming process of attaching and taking measurements from several electrodes. Not only does the test preparation and locating of electrodes require some expertise, so does recognition as to whether a sufficient signal is being detected for analysis to be performed. Even under the best of circumstances, the signal-to-noise ratio (SNR) of AER is low.

Yet a further disincentive to clinical use of dyslexia AER testing is the fact that a desired test population are newborn infants. Known electrode attachments and analysis equipment are cumbersome, imposing in appearance, with long and potentially dangerous wiring harnesses, tending to disconcert parents and other visitors to maternity wards who may witness the test.

Consequently, a significant need exists for an AER (e.g., dyslexia) testing device and method that is suitable for widespread clinical use.

### Brief Summary of the Invention

The invention overcomes the above-noted and other deficiencies of the prior art by providing a screening device that is simple to use in a clinical environment. A headset is readily engageable to a head of an infant subject and positions an electrode on a reference location on the head, such as the cheek or forehead or other portion, and positions a signal electrode advantageously with regard to the infant's ear. This signal electrode is thus readily positioned proximate to the auditory processing locations on the infant's head to sense an Auditory Evoked Response (AER) after an auditory stimulus is given to the infant. Simplified electrode placement allows clinical use by those without having specific neurological training.

In one aspect of the invention, the headset device positions the electrodes for convenient data acquisition and further stores the AER data after the auditory stimulus for later uploading via a communication link to a data analyzer. Thereby, the expense of a data analyzer is removed from the device, allowing one data analyzer to be more efficiently used to support a large number of devices. Moreover, the headset device is more portable and less intrusive for use in various clinical settings.

These and other objects and advantages of the present invention shall be made apparent from the accompanying drawings and the description thereof.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIG. 1 is a perspective view of an integrated Auditory Evoked Response (AER) headset for clinical screening for AER mapping (e.g., dyslexia) in infants.

FIG. 2 is a close-up view of an electrode attached to one of the headset's flexible arms.

FIG. 3 is a perspective view of the AER headset of FIG. 1 with one clamshell cover removed.

FIG. 4 is a top view of a flex-circuit electronic harness for the AER headset of FIG. 1.

FIG. 5 is a functional block diagram of a controller of the AER headset of FIG. 1.

FIG. 6A is a top diagrammatic view of a recurved frame and earpieces of the AER headset of FIG. 1, shown in a relaxed position.

FIG. 6B is a top diagrammatic view of the recurved frame and earpieces of the AER headset of FIG. 6A, shown in an expanded position.

FIG. 7 is a block diagram of the AER headset of FIG. 1 as part of an auditory evoked response mapping system.

FIG. 8 is a flowchart describing a procedure or sequence of operations performed by the AER headset of FIG. 1 to stimulate, capture, and analyze EEG's.

FIG. 9 is a graph of EEG auditory evoked response as a function of time illustrating a screening schema for AER analyses such as peak latency, cognitive functional significance, cortical distributions, and component brain sources.

FIG. 10 is a table of a stimulus library maintained and utilized by the auditory evoked response mapping system and/or the AER headset of FIG. 7.

FIG. 11 is a diagram of an illustrative AER protocol for audiometry, mismatch negativity, and equal probability testing performed as part of the auditory evoked response mapping system of FIG. 7.

### Detailed Description of the Invention

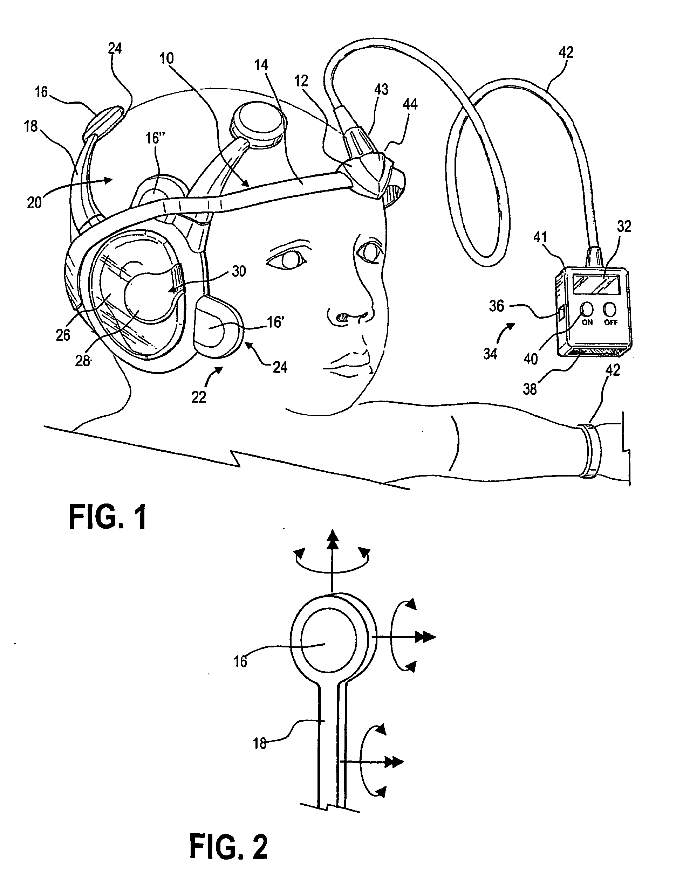

In the drawings where like members are given the same reference numeral, in FIG. 1, an integrated Auditory Evoked Response (AER) headset 10 includes embedded features that enable clinicians to readily perform an electroencephalogram (EEG) test without the necessity of extensive training. Portability of diagnostic data taking allows use whenever and wherever desired. Economy of use is achieved by centralized processing of the diagnostic data so that a great number of headsets 10 may be used without the necessity of expensive waveform processing equipment at each location. Collecting data from many screened individuals enables enhanced and improved diagnostic algorithms to be created and implemented. Furthermore, the headset 10 includes features that speed its use while avoiding human error and the need for extensive training.

To these ends, the headset 10 incorporates a control module 12 that advantageously allows the headset 10 to be portable and to be used in a clinical setting by including pre-loaded or downloadable testing protocols managed by the control module 12, enhancing ease of use. The headset 10 further includes an elastic, semi-rigid frame 14, which contains the control module 12. In particular, the frame 14 automatically positions six conductive electrode plugs ("electrodes") 16 via flexible arms 18 to specific positions 20 relative to the subject's ears correlating to portions of the brain responsible for auditory processing. These flexible arms 18 are advantageously cantilevered to exert a force upon the electrodes 16 to assist in obtaining good electrical contact with the subject's skin. In the illustrative embodiment, this alignment is assisted by the recurved frame 14 oriented to pass over the forehead. This convenient positioning greatly simplifies the generally accepted practice of manually positioning each electrode on the scalp in reference to a central point. A similar reference electrode plug 16' is positioned by flexible arm 18' to a forehead location 22 of the subject, this point selected for being relatively at an electrical ground potential relative to the auditory processing locations and for being readily accessible with a supine subject.

Each electrode plug 16, 16' contacts the subject's skin via an electrode pad 24, 24' that includes electrical contacts to pick up the voltage signal of the AER. The frame 14 and flexible arms 18, 18' exert a force respectively upon each electrode plug 16, 16' and electrode 24, 24' to achieve a good electrical contact. Each electrode pad 24, 24' may be individually replaceable to ensure proper operation and/or sterilization requirements. Alternatively, a larger portion of the headset 10 may be replaceable for such reasons. Yet a further alternative may be that the electrodes 24, 24' may be compatible with sterilizing agents, such as an alcohol wipe. The electrode pads 24, 24' may support or incorporate an electrically conductive substance such as saline to enhance electrical contact. Alternatively or in addition, the electrode plugs 16, 16' and electrode pads 24, 24' may incorporate a pneumatic seal when manually depressed against the subject's skin, or even further include an active pneumatic suction capability to achieve good contact.

Fluid-filled bladders (not shown) may be advantageously incorporated into portions of the headset 10, such as inside the ear cups and electrodes, in order to provide a uniform contact with the subject's head, reducing discomfort and the likelihood of impedance variations. Alternatively, a resilient material (e.g., foam, gel) may be used instead of fluid-filled bladders.

An exemplary electrode 24, 24' may employ an active digital electrode approach for incorporation into the headset 10 to address the need for sensitivity, enhanced signal to noise performance, and economy, described in greater detail in the afore-mentioned patent application entitled "ACTIVE, MULTIPLEXED DIGITAL NEURO ELECTRODES FOR EEG, ECG, EMG APPLICATIONS".

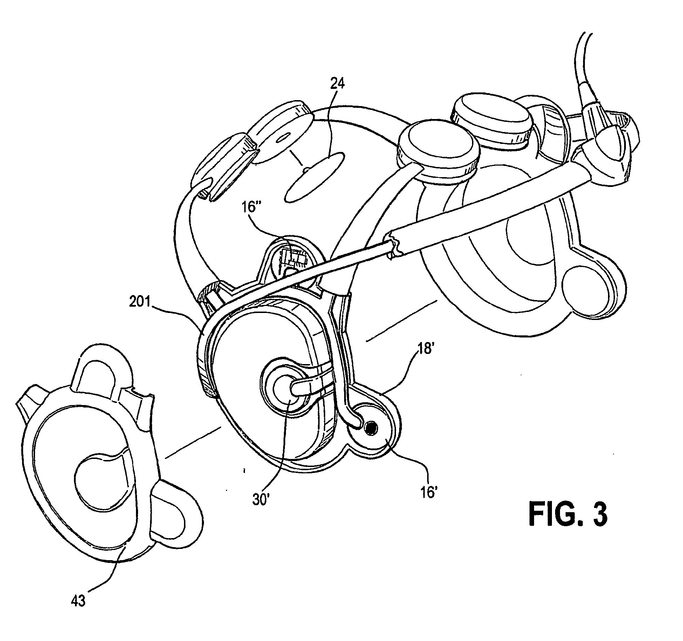

In FIGS. 1 and 3, the frame 14 also supports ear cups (earpieces) 26 that position sound projectors 28 in front of the respective subject's ear. The headset 10 includes a speaker 30 for each ear that generates an auditory signal in response to an electrical signal from the control module 12. Each speaker 30 may be in a respective ear cup 26. Alternatively, each speaker 30 may be proximate to the control module 12, such as a piezoelectric transducer, that generates a sound that is directed through a pneumatic sound tube (not shown) to the sound projector 28 in the ear cup 26. This latter configuration may have advantages for having a replaceable ear cup assembly wherein active components are relegated to a reusable portion or where the active components are externally coupled to a passive, perhaps disposable headset. An electrode (not shown) may advantageously be included in the ear cup 26 for ensuring location caudad to the sylvan fissure.

When the headset 10 is used, simplified indications and controls 32 let the clinician know that the headset 10 is operational. For instance, an indication may be given that sufficient battery power exists, that the electronic components have passed a built-in test, etc. Thereby, the clinician, even with little specific training into the AER waveform analysis, is able to readily perform the data acquisition on the subject.

Although the headset 10 may include all of the functionality required to perform a (e.g., dyslexia) AER testing protocol, the headset 10 advantageously accepts an external electrical connector 34 at an interface 36 so that additional functionality may be selectively used. For instance, rechargeable batteries (not depicted in FIG. 1) in the headset 10 maybe charged. The interface 36 may accept subject identification information to be linked with the diagnostic data taken. For instance, a personal computer, personal digital assistant, or a keyboard may be interfaced to the headset 10 as a means to input subject identification information. An illustrative input device, depicted as an identity scanning device 38, such as the OPTICON PN MSH-LVE4100 barcode scanner module integrated into the control box 41, is activated by a push button 40 presented upon a control box 41 to read a patient identification band 42. The illustrative identity scanning device 38 advantageously has a short reach via cable connection 43 to minimize the likelihood of misidentifying the subject being tested. The identity scanning device 38 may advantageously sense alternatively or in addition to barcodes other indicia of identity, such as by passive radio frequency identification (RFID) (e.g., PHILIPS PN HTRM440), fingerprint scanning, or manual keypad entry via an input device coupled or attached to a control box. Furthermore, such control box functions may be integrated into the headset rather than being tethered thereto.

It should be appreciated by those skilled in the art having the benefit of the present disclosure that a hard-wired interface 36, such as a Universal Serial Bus (USB) interface, may be used as depicted or a wireless connection may be made, such as using the BLLTETOOTH standard or other type of link.

Furthermore, a barcode identifier may be a one-dimensional or a two-dimensional barcode. Similar, the identifying information may be in the form of an embedded radio frequency (RF) target that puts off a unique return when energized by an RF carrier signal. Other types of identifying information may be used consistent with aspects of the present invention.

FIG. 2 depicts the flexible arm 18, 18' supporting the electrode plug 16 annotated to denote resilient characteristics inherent so that a good electrical conduct is achieved. It will be appreciated that wiring or conductive ink applied to or formed therein may be used to electrically couple the electrode plug 16, 16' to the control module 12.

In FIG. 3, the electrodes 16 and 16', along with the speaker 30 are captured in a clamshell cover 43. One or more active electrodes 16 may be a high frequency electrode which has been set to capture brainwaves at around 20,000 Hz. Disposable electrode contact pad 24, shown detached, may be impregnated with an electrolytic gel to lower impedance. This headset 10 includes three different types of electrodes. High frequency electrodes 16, reference electrodes 16' at the patient's cheek, and low frequency electrodes 16". As mentioned before, some electrodes 16 advantageously achieve good electrical contact via cantilevered flexible arms 18 while those closely coupled to the ear cups 26 receive a similar inward force from the recurved frame 14.

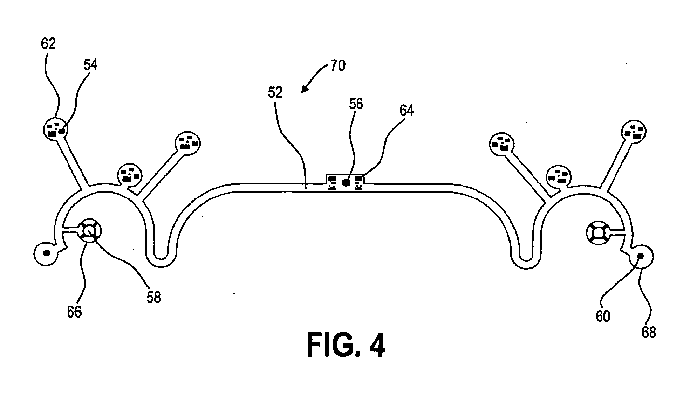

In FIG. 4, a flex-circuit electronic harness 50 is depicted as an economical fabrication approach with the electrodes 16, 16', interconnects, and other headset electronics integrated onto a flexible printed circuit 52. Electrode electronics 54, control electronics 56, earpiece electronics 58, and electrode pad connectors 60 are electrically connected to flexible printed circuit 52 at flexible circuit areas 62, 64, 66, 68 respectively. Thus, an advantageous flex-circuit electronic harness 50 lends itself to being shaped to a subject's cranium and to being exteriorly cantilevered into good electrical contact with the subject's skin.

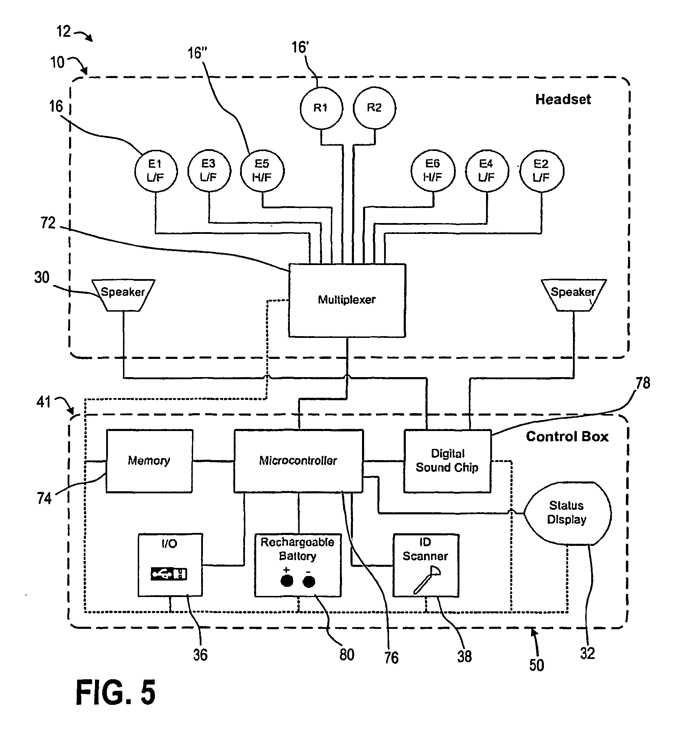

FIG. 5 depicts an illustrative control module 12 of the headset 10 formed as an electronic circuit 70. It should be appreciated that the electronic circuit 50 may advantageously be produced in large-scale production as a custom Application Specific Integrated Circuit (ASIC) wherein all or many of these and other functions are incorporated into a single silicon wafer.

In the illustrative version, a number of discrete devices are used to perform the acquisition of AER data. The electrodes 16, 16' produce a low voltage signal that is selectively transmitted to the control box 41 by a multiplexer 72. At least one electrode 16, 16' may advantageously be designed for high frequency data capture (e.g., typical sampling rate of 20,000 Hz) and/or at least one electrode may be designed for low frequency data capture (e.g., typical sampling rate of 250 Hz). The gain, filters, and A/D conversion settings may thus be different to accommodate the differences in signal characteristics. In particular, the high frequency electrode(s) may be used to capture low amplitude, high frequency brainwaves as in auditory brainstem response (ABR) testing for hearing defects. The low frequency electrode(s) may be used to capture higher amplitude, lower frequency brainwaves like the middle latency response (MLR) and the late latency response (LLR or slow-wave). These waves are commonly used to detect auditory processing disorders (APD), attention deficit disorder (ADD), and dyslexia.

The multiplexed signal therefrom is received by an integrated memory 54, such as a TOSHIBA, Part. No. TC58128AFT, 128 MB 3.3V Flash Memory in a 48 Thin Small-Outline Package (TSOP) Surface-Mount Technology (SMT) package. The memory 74 receives input data from external devices, such as the barcode scanner 38 via the interface (e.g., USB port) 36. The memory 74 is also preloaded or uploaded with a testing protocol and stores a number of testing session data records so that the headset 10 may be repeatedly used prior to uploading results.

The processing is performed by a microcontroller 76, such as MICROCHIP PIC16C765-I/PT, which advantageously includes analog-to-digital (A/D) Converters and USB Communication capability. An example of the processing includes sending a predetermined number of audio signals of a predetermined pitch, volume and duration or a previously recorded and digitized sound, and recording the resultant AER waveform. In particular, the microcontroller 76 may communicate with the multiplexer 72 to control which electrodes 16, 16', 16" are being sampled. The electrodes can be turned on and off in a serial fashion to capture early, high frequency waves and later, low frequency waves evoked from the same initial stimulus. This will produce optimized signal detection with a minimum of file size.

The desired audio signals are produced by a digital sound card 78, such as by WINBOND ELECTRONICS, ISD4002-150E, "Single-Chip Voice Playback Device" that produces the audio signals on speakers 30. The electronic circuit 70 is powered by a power supply 80, such as an ULTRALIFE UBC502030, Rechargeable 200mAh battery.

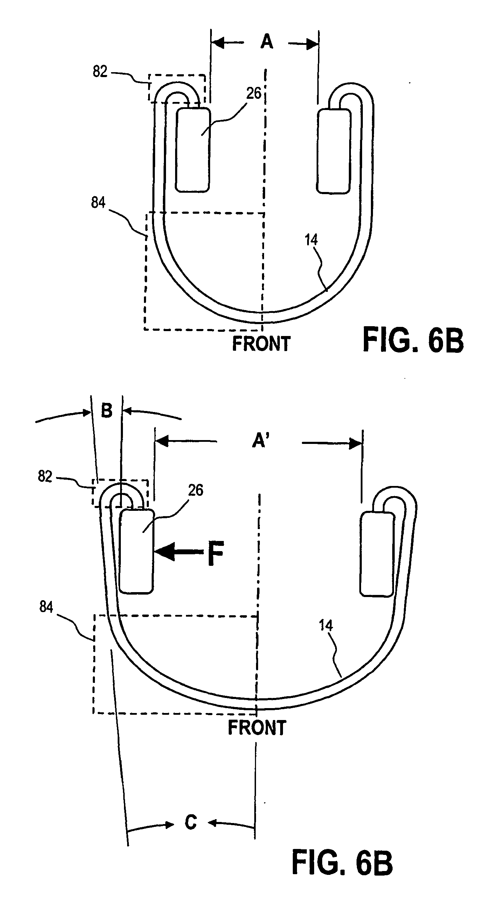

In FIGS. 6A-6B, headset frame 14 is recurved such that when a force F is applied, as when there is a need for the headset 10 to be installed on a large head increasing the distance from A to A', the bending angle B of the ear cup 26 in the general area 82 aft of the ear cup 26 of the headset frame 14 is equal to the bending angle C of the headset frame 14 in the general area 84 forward of the ear cup 26. This will keep the orientation of the left and right earpieces 26, with respect to the subject's ears, the same for a broad range of head sizes.

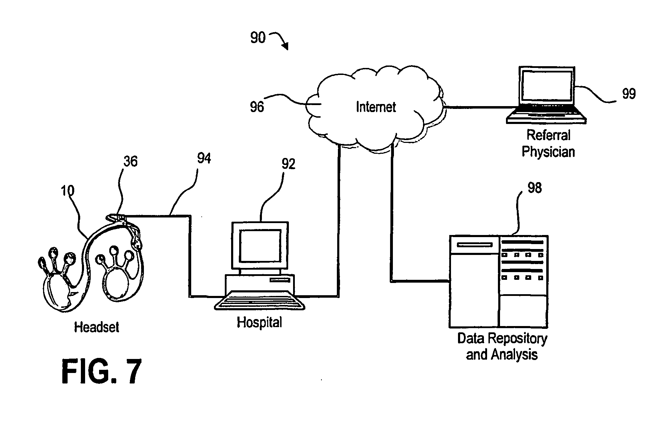

FIG. 7 depicts an AER (e.g., dyslexia) screening test system 90 that advantageously provides for economical testing, billing, long-term data storage and analysis for analysis refinement, subsequent therapeutic measures, and other features. To this end, the headset 10 may be in electrical communication with a hospital system 92 via a cable or wireless link 94 so that accomplishment of the dyslexia screening test is noted for patient health records and for billing records. Also, the hospital system 92 may facilitate communication across a network, such as the Internet 96, to a remote processing facility, depicted as a data repository and analysis computer 98.

This data repository and analysis computer 98 allows for the most up-to-date waveform recognition techniques to be employed to diagnose a dyslexia condition. Moreover, the computer 98 may process a number of data from screening tests to make such analysis more cost effective. Moreover, historical data may be mined as recognition techniques improve to capture previously undiagnosed conditions or to otherwise correlate previous test results with other forms of data to further refine the diagnostic process. It should be appreciated that the analysis performed by the data repository and analysis computer 98 could further include neural net processing, wherein the neural net is trained to recognize a waveform characteristic of dyslexia or other conditions.

Positive, inconclusive, and/or negative screening test results may be forwarded to an appropriate recipient, such as a referral physician 99 for further diagnostic testing and/or therapeutic measures.

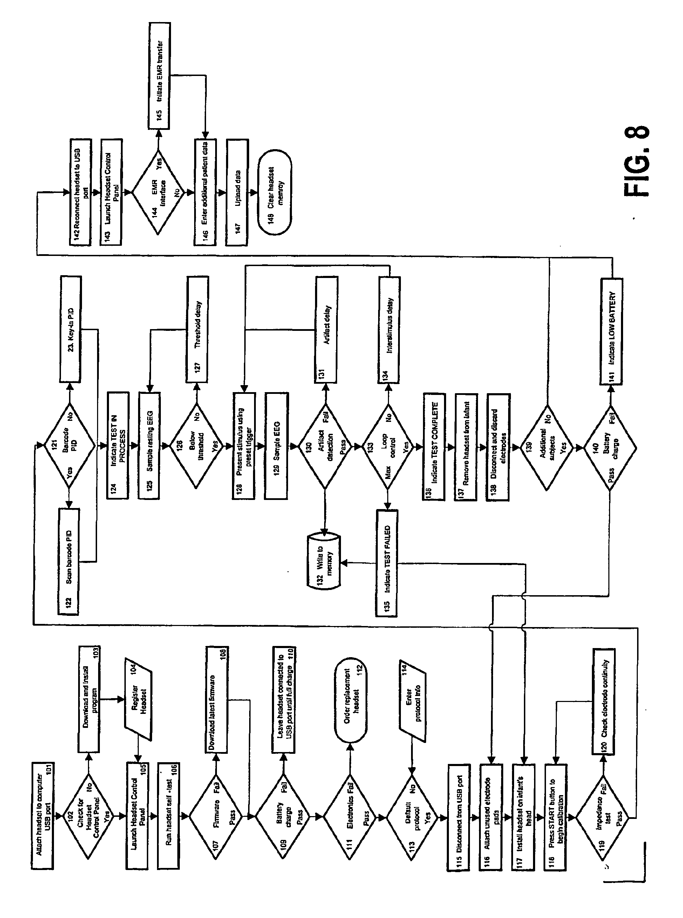

FIG. 8 depicts an illustrative procedure or sequence of operations 100 for AER (e.g., dyslexia) screening performed by the test system 70 of FIG. 7. In block 101, the headset is attached to a computer USB port. If determined that the headset control panel indicates the need for initializing the headset (block 102), then a headset program is downloaded and installed (block 103) and the headset identification number and initialization status is registered (block 104). If initialization is not needed in block 102 or after registering in block 104, then the headset control panel is launched (block 105) and a self-test is performed by the headset (block 106). If the firmware is determined to have failed (block 107), then the latest firmware may be downloaded (block 108). If the battery is determined to have failed a charge test (block 109), then the headset is left connected to the USB port until fully charged (block 110). If the electronics self-test fails (block 111), then an indication is given to the user or electronically transmitted back via the USB port to order a replacement headset (block 112). If the user inputs that default protocol is not to be used (block 113), then the headset receives protocol information from the user, perhaps input through the control box or from a PC interface (block 114). In block 115, the headset is disconnected from a hospital computer or other device after a previous upload of screening test data, download of an updated test protocol, and/or charging of the batteries in the headset. The headset is prepared for the next subject by ensuring that the headset is sterile and has operable electrodes. One way is as depicted in block 116 by attaching an unused electrode pad to each of the electrode arms.

With the headset ready, the headset is placed upon an infant subject's head. The frame of the headset simplifies placement by including ear cups and a forehead frame to be aligned with the subject's eyebrows that intuitively guide the clinician in proper placement (block 117). This includes properly positioning reference electrodes at the patient's cheeks, although other predetermined reference locations may be selected, such as the forehead. Simplified initiation of the test is provided by depressing the start button on the attached control box (block 118). The headset interprets this button push and initiates a self-test to verify good reception of an EEG signal from the subject (e.g., impedance test) (block 119). The self-test is indicated on the headset indicator LED lights or control box. If failed, the clinician removes the headset from the infant's head and checks electrode continuity (block 120), which may entail visually checking for good electrode contact and/or reconnecting the headset to a hospital device to evaluate the cause of the failure (block 120). For instance, the headset may provide a more detailed explanation of the failure over the interface.

If in block 108 the self-test was deemed a pass, then a determination is made as to whether a machine readable patient identification (PID) such as a barcode is available (block 121). If so, the clinician uses the scanning device to scan in a PID code from the subject (block 122), else the PID is manually keyed in (block 123). The headset responds by giving an indication of a test in process so that the clinician leaves the headset undisturbed (block 124). Then, the headset samples resting EEG at the various electrodes (block 125), This sampling includes making a determination whether an EEG voltage is below a threshold indicative of a resting, unstimulated state (block 126), and if not, a threshold delay is imposed (block 127), looping back to block 125. Else, if the appropriate initial condition is found in block 126, then a stimulus is presented using a preset trigger defined by the protocol (block 128). The EEG is then sampled at the appropriate combination of electrodes and at a sample rate appropriate for the frequency of interest (block 129).

Another feature that may enhance consistent results is defining an initial starting point on the same slope of a detected resting brainwave (e.g., rising slope, falling slope, apex, nadir).

Advantageously, the headset performs a data integrity check, such as by comparing the sampled data against various criteria to detect artifacts indicative of noise or external stimuli that corrupted the data sample (block 130). If detected, then an artifact delay is imposed (block 131) before looping back to block 128. Else, the data samples are written to memory in the headset (block 132), including storing the PID for tagging to the screening test data. Typically, the test protocol includes a series of stimuli and samples. Thus, a determination is made that another control loop is to be performed (block 133). If so, an appropriate interstimulus delay is imposed to return to a resting EEG (block 134) followed by looping back to block 128. However, if more control loops are warranted but a threshold is exceeded for a maximum time or a maximum number of attempts, then the test failed indication is given (block 135) and the procedure returns to block 117 for the clinician to reposition the headset for retesting. If, however, in block 133 the inner and outer control loops that define the testing protocol are deemed complete, then a test complete indication is given to the clinician (block 136), such as by illuminating an appropriate LED light.

If test complete is determined in block 136, then the headset is removed from the infant subject's head (block 137) and the used electrode pads are removed and discarded from the headset (block 138). If another subject is to be tested prior to uploading screening test data (block 139), a battery charge check is made (block 140) to see if the remaining charge is sufficient. If it passes, then processing loops back to block 116 to prepare the headset for the next subject. If failed, then a low battery indication is given (block 141).

If no additional subjects are determined in block 139 or if low battery is determined in block 141, it is time for reconnecting the headset to the USB port of the hospital computer (block 142), which recharges the headset and also provides an opportunity to activate an Internet connection to initiate data upload and any new test protocol download. In particular, a headset control panel is launched for interacting with the clinician (block 143). If an electronic medical record (EMR) interface is determined to be available (block 144), then an EMR transfer is initiated (block 145). If EMR transfer is not available or after EMR transfer is initiated, then the clinician is afforded an opportunity to enter additional patient data (block 146). The data is uploaded to the AER system (remote user) for analysis and disposition (block 147)

For instance, the remote user may perform diagnostic analysis on the received screening test data to see if the AER data is indicative of dyslexia. If a determination is made that the results are not positive for dyslexia, then the appropriate recipient is informed, such as the parent or the attending pediatrician or obstetrician. If positive, then the test results may be advantageously forwarded to an in-network referral physician, such as a child psychologist.

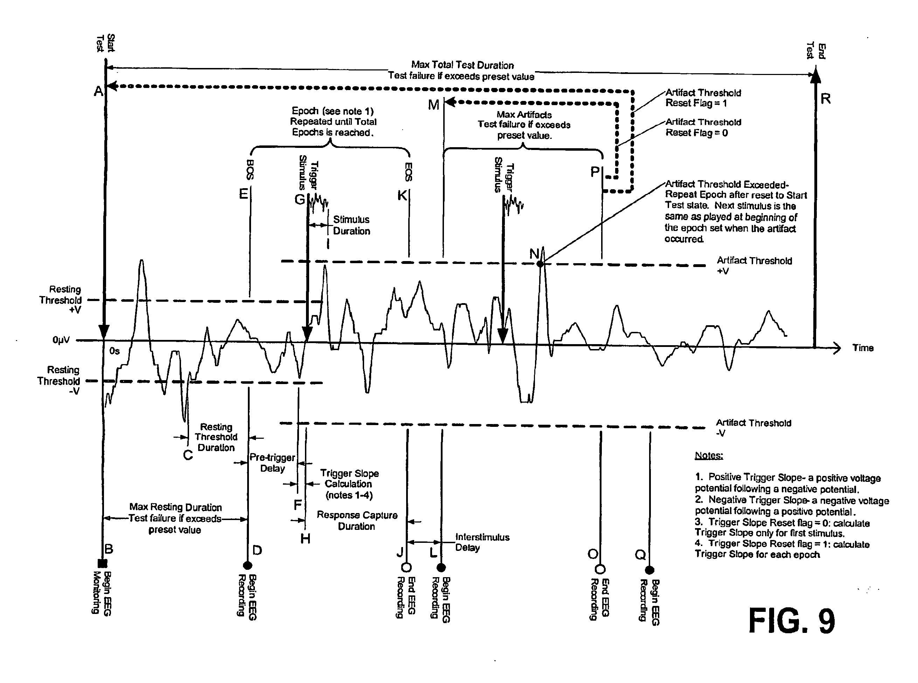

In FIG. 9, a timing chart illustrates a sequence of events involved in an AER test. At time "T_{A}" the test subject's barcode wristband is scanned and the test begins. Concurrently, the headset begins monitoring the brainwaves at time "T_{B}" to identify when the amplitude of the resting brainwaves falls below the preset resting threshold at time "T_{C}" and remains there for a preset duration. This begins the recording of the brainwaves at time "T_{D}". This point is called beginning of series (BOS), at time "T_{E}". Next, the headset calculates the slope of each subsequent brainwave at time "T_{F}" and triggers the stimulus when the slope criteria is met at time "T_{G}" beginning a response capture period at time "T_{H}". The stimulus is generally of short duration at time "T_{I}". At the end of the response capture period, the brainwave recording stops at time "T_{J}". For a single stimulus series, this is called end of series (EOS) at time "T_{K}". Time sequence from time "T_{E}" to time "T_{K}" defines the first epoch. A predetermined interstimulus delay passes at "T_{L}" before the next epoch at time "T_{M}" is begun. During the next epoch, the chart shows an artifact where the amplitude of the recorded brainwave exceeds the artifact threshold at time "T_{N}". At the end of this epoch the EEG recording stops at time "T_{O}" and the sequence is redirected at time "T_{P}" back to the beginning of series at time "T_{M}" if the artifact threshold reset flag is set to "1" or to before the resting threshold at time "T_{A}" if the flag is set to "0". If the artifact threshold is not exceeded, a new epoch is begun at time "T_{Q}". The test ends when all epochs are completed or when the total test time is exceeded at time "T_{R}".

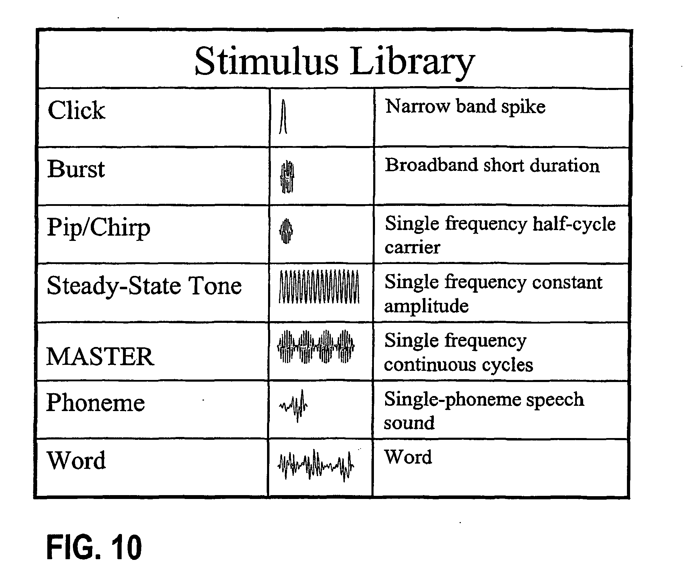

In FIG. 10, an illustrative stimulus library is depicted having seven general types of stimulus, representing the kind of stimuli that can be downloaded into headset memory to be used to evoke a brainwave response. Any recorded, or synthesized audio stimulus may be used with this list being merely exemplary. In particular, the library may include a click that is of a narrow frequency band of extremely short time duration (i.e., spike), a burst that is a broadband signal of short duration.

In Table 1, an illustrative configuration table lists data capture settings that may be accessed, selected, modified, or otherwise utilized by the headset 10 to adapt its testing capabilities. For instance, a range of preset electrode locations may be configurable, for example 10 to 20 locations identified by an electrode location label. For instance, a selected headset 10 with its choice of cantilevered arms and electrode placements may use a subset of available locations. However, the system is capable of being used with different locations. Data capture start and end defines what latency is expected for the brainwave of interest. Signal gain sets amplification as appropriate for the particular electrode location, brainwave of interest, and perhaps a detected impedance / resting brainwave pattern. In addition, artifact detection parameters may be advantageously incorporated so as to determine if a particular AER test did not receive an undisturbed result This artifact detection may be a voltage threshold that should not be exceeded during the data sampling.

| **TABLE 1** | **Data Capture Settings** |
|---|---|
| Electrode Location | Location I.D. using 10-20 system |
| Electrode Selection | Which electrodes will be selected for data capture |
| Data Capture Start-End | When should the data capture begin and end |
| Data Capture Rate | At what rate should the system sample the electrodes to capture data |
| Signal Gain | Signal amplification |
| Artifact Threshold | Voltage threshold to be used to instruct the system when to replay the stimulus set |

Table 2 represents the kind of sequences that can be downloaded into the headset memory to be used to evoke a brainwave response. User-defined sequences may also be used.

| **Table 2** | **Sequence Library** |
|---|---|
| Repetition | Repeat single stimuli |
| Steady-State | Single tone with long duration |
| Equal Probability | Multiple stimuli repeated, each repeated an equal number of times |
| Oddball Paradigm (MMN): | Single standard stimuli with one or more deviant stimuli |
| Variable Frequency | Constant volume, vary frequency |
| Variable Volume | constant frequency, vary volume |
| Variable Time Warp | Constant tone, vary duration |
| User Defined | User defined sequence presentation, volume, tone, and time warp |

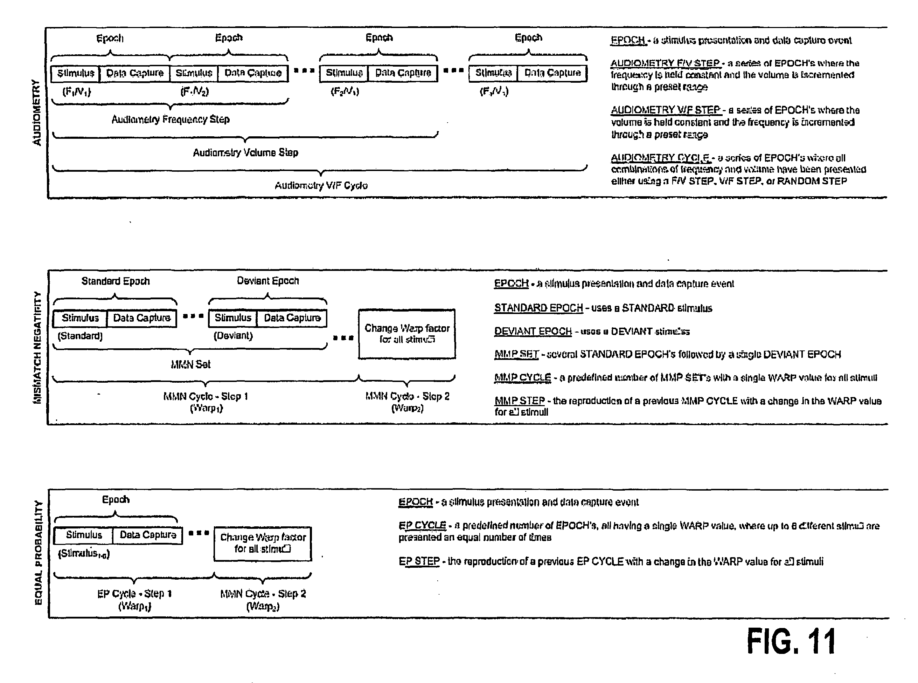

In FIG. 11, the integrated AER headset 10 performs a selected or predefined protocol that detects an AER for mapping, illustrated by a protocol that includes audiometry, mismatch negativity, and equal probability testing such as may be performed as part of the procedure 100 of FIGS. 8 and 9. The headset 10 references characteristics of each epoch, which includes a stimulus presentation and a data capture event, to be performed within an audiometry frequency step. One or more audiometry frequency steps in turn comprise an audiometry volume step. One or more audiometry volume steps in turn comprise an audiometry volume/frequency cycle. Thus, the audiometry frequency/volume step combination holds a selected increment of frequency constant while stepping through volume increments before moving onto a different frequency increment. Thereby, the subject's hearing sensitivity at various frequencies is determined.

Variations in the audiometry cycle may be selected. For instance, an audiometry volume/frequency step combination entails a series of epochs wherein the volume is held constant and the frequency is incremented through a preset range before moving onto another increment of volume. As another example, an audiometry random step combination performs each combination of frequency and volume increment but in a random order.

In a mismatch negativity (MMN) set, several standard epochs are included with a deviant epoch having a deviant stimulus. A predefined number of MMN sets are performed with a single warp value for all stimuli to perform an MMN cycle. Then, another MMN step is performed as a reproduction of the previous MMN cycle but with a change in the warp value for all stimuli.

In an equal probably (EP) screening, an EP cycle includes a predetermined number of epochs, all having a single WARP value wherein up to 6 different stimuli are presented an equal number of times. An EP step is a reproduction of a previous EP cycle with a change in the WARP value for all stimuli.

In use, a headset 10 advantageously integrates sound projectors (earphones) 28 and flexible electrode arms 18, 18' that easily and accurately position electrodes 16 on a patient's scalp. A recurved headset frame 14 ensures the proper angle between ear cups 26 as well as providing a convenient ability to position the headset with a supine subject at the brow of the subject. Flex circuitry incorporates networked electrodes within an economical assembly. The contact points of the headset 10 may advantageously include fluid-filled bladders that provide comfort, a good seal for excluding noise from ear cups 26, and uniform impedance at electrodes 16. A digital control box 41 contains a microprocessor, battery, and a patient ID system (e.g., barcode or RF1D scanner) in order to perform the auditory testing conveniently in a clinical setting. Samples are taken from each electrode 16, 16', 16" at an appropriate data rate for the appropriate frequency and duration to reduce data storage file size. Automatic detection of artifacts causes replay of affected epochs to avoid failed tests. Different audio tests (e.g., audiometry, mismatched negativity, equal probability) are supported by a PC-based programming system that connects to a web-based database for downloading/modifying testing protocol configurations for loading onto a headset. A particularly advantageous protocol is supported by randomizing stimulus sequences, which is used when presenting multiple stimuli when each needs to be repeated an equal number of times in random order. Data integrity is maintained by performing artifact detection and resting threshold monitoring before initiating stimulus based upon the slope of the resting brainwaves.

By virtue of the foregoing, an easy to use headset 10 allows testing one or more subjects even when not conveniently near to outlet power or data network access points. Training of clinician personal is simplified by having protocols automatically set up as well as built-in hardware and data integrity tests.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art.

For example, although a headset 10 and distributed dyslexia screening test system 70 have been illustrated that have certain advantages, all of the functionality may be incorporated into a headset. Alternatively, a disposable headset may be used with most of the active components and processing connected thereto. As yet a further alternative, a general-purpose computer may be configured to perform the testing protocol and/or the waveform analysis with the headset including essentially only electrodes and speakers.

As another example, although screening of infants is advantageously emphasized herein, older children and adults may be advantageously tested as well.

As yet an additional example, although dyslexia is a condition discussed herein, it will be appreciated that other neurological conditions may advantageously be tested by a similar headset with a frame positioning electrodes in a desired position and configuration. Examples include autism, hearing loss, schizophrenia, etc.

## Claims

1. A screening device, comprising:
a frame (14) shaped to be engageable to a head between a reference location, at least one ear and a signal detection location;
a reference electrode (16') attached to the frame at the reference location;
a signal electrode (16') attached to the frame at the auditory processing location;
an auditory signal producer (28, 30) positioned by the frame (14) over the ear; and
an auditory evoked response (AER) data processor (12, 41) operably configured to initiate an auditory signal from the auditory signal producer (28, 30) and to perform a signal processing operation on an AER signal sensed across the reference and signal electrodes (16, 16'):
**characterized by**:
a data analyzer (41, 98) operably configured to characterize the AER signal and to compere the characteristics to at least one predetermined AER characteristic, wherein the at least one predetermined AER characteristic is associated with a neurological condition.

2. The screening device of claim 1, further comprising a cantilevered flexible arm (18) connecting the signal electrode (16) to the frame (14).

3. The screening device of claim 1, further comprising a second signal electrode (16") attached to the frame (14).

4. The screening device of claim 3, further comprising a multiplexing channel controlled by the AER data processor (12, 41) for selectively sampling the first and second signal electrodes (16, 16")

5. The screening device of claim 3, wherein the AER data processor (12, 41) is further operatively configured to sample the first signal electrode (16) at a low frequency sampling rate and to sample the second signal electrode (16") at a high frequency.

6. The screening device of claim 5, further comprising a multiplexing channel controlled by the AER data processor (12, 41) for selectively sampling the first and second signal electrodes (16, 16").

7. The screening device of claim 3, further comprising a flexible printed circuit harness (50) containing the electrodes (16, 16', 16") and communication paths to the AER data processor (12, 41) and shaped for conforming to the head under the resilient urging of the frame (14).

8. The screening device of claim 1, further comprising a test subject identification device (38), the AER data processor (12) further operably configured to associate a test subject identification with the AER signal.

9. The screening device of claim 8, wherein the test subject identification device (38) comprises a barcode scanner.

10. The screening device of claim 8, wherein the test subject identification device (38) comprises a radio frequency identification scanner.

11. The screening device of claim 1, wherein the at least one predetermined AER characteristic comprises a dyslexic AER characteristic.

12. The screening device of claim [[11]] 1, further comprising a communication link (43), wherein the data analyzer (41, 98) is coupled to the frame (14) via the communication link (43).

13. The screening device of claim 1, wherein the AER data processor (12, 41) comprises a control module (12) integral to the frame (14).

14. The screening device of claim 1, wherein the frame (14) includes a disposable portion that includes the electrodes (16, 16', 16").

15. The screening device of claim 1, wherein the AER data processor (12, 41) includes digital storage (74) configured to store the AER data.

16. The screening device of claim 1, wherein the AER data processor (12, 41) is further operably configured to perform a sequence of screening tests, and to store in the digital storage (74) AER data associated with each test.

17. The screening device of claim 16, wherein the digital storage (74) further includes a predetermined test protocol.

18. The screening device of claim 1, wherein the AER data processor (12, 41) is further operably configured to generate a user indication of a test condition.

19. The screening device of claim 1, wherein the frame is operably shaped to connect between the ears across a front portion of a patient's head.

20. The screening device of claim 1, wherein the frame (14) comprises a recurved frame (14) and a pair of ear cups (26) attached to each end thereof.

21. The screening device of claim 1, wherein the frame (14) comprises an ear cup (26) having a resilient portion inwardly affixed thereto.

22. The screening device of claim 1, wherein the frame (14) further comprises an ear cup (26) having an electrode (16") registered caudad to the sylvan fissure of a subject.

23. A method of performing auditory evoked response (AER), comprising:
positioning a device (10) on the head of a subject, the device positioning a sound producer (28, 30), a reference electrode (16') and a signal electrode (16);
generating an auditory stimulus;
recording AER data across the reference and signal electrodes;
**characterized by**:
connecting the device to a data analyzer (41, 98):
characterizing the AER data with the data analyzer (41, 98); and
comparing the AER data characteristics to at least one predetermined AER characteristic with the data analyser (41. 98), wherein the at least one predetermined AER characteristic is associated with a neurological condition.

24. The method of claim 23, wherein recording the AER data further comprises:
storing the AER data on the device (10); and
transmitting the stored AER data to the data analyzer (41, 98).

25. The method of claim 23, wherein positioning the device (10) on the head of the subject further comprising positioning the subject face up and positioning the device (10) across a forward portion of the subject's head.

26. The method of claim 23, wherein generating the auditory stimulus further comprises:
in response to determining the sensed electrode voltage exceeding a threshold, imposing a sampling delay in pursuit of a resting brain state.

27. The method of claim 23, wherein generating the auditory stimulus further comprises:
detecting a resting brain wave; and
initiating the auditory stimulus at a predetermined slope of the resting brain wave.

28. The method of claim 23, further comprising:
in response to determining the AER data to contain an artifact, imposing a sampling delay and repeating an epoch of auditory stimulus and sampling AER data.

29. The method of claim 23, further comprising:
accessing a remotely stored auditory testing protocol into the device (10); and
disconnecting the device (10) prior to positioning a device (10) on the head of the subject.

30. The method of claim 23, wherein the device (10) positions the reference electrode (16') a low frequency signal electrode (16") and a high frequency signal electrode (16), the method further comprising sampling the low frequency signal electrode (16") at first sampling rate and sampling the high frequency signal electrode (16) at a higher second sampling rate.

31. The method of claim 23, wherein the device (10) positions the reference electrode (16'), a first signal electrode. (16) and a second signal electrode (16"), and wherein sampling the first and second frequency signal electrodes (16, 16") further comprises for each electrode (16.16"):
sensing an EEG voltage;
converting the sensed voltage to a digital value;
sampling the digital value at a predetermined sampling rate over a multiplexing channel; and
recording the multiplexed digital data.

32. The method of claim 31, wherein sampling the digital value at a predetermined sampling rate over the multiplexing channel comprises further comprises sampling the first signal electrode (16) at low frequency and sampling the second signal electrode (16") at a high frequency.

33. The screening device of claim 1, wherein the at least one predetermined AER characteristic includes at least one characteristic selected from the group consisting of a dyslexia AER characteristic, a autism AER characteristic, a schizophrenia AER characteristic, and an attention deficit disorder AER characteristic,

34. The method of claim 23, wherein the at least one predetermined AER characteristic includes at least one characteristic selected from the group consisting of: a dyslexia AER characteristic, a autism AER characteristic, a schizophrenia AER characteristic, and an attention deficit disorder AER characteristic.

## Patentansprüche

1. Screening-Vorrichtung, die Folgendes umfasst:
einen Rahmen (14), der so geformt ist, dass er einen Kopf zwischen einem Referenzort, mindestens einem Ohr und einem Signaldetektionsort in Eingriff nehmen kann;
eine Referenzelektrode (16'), die an dem Rahmen an dem Referenzort angebracht ist;
eine Signalelektrode (16), die an dem Rahmen an dem Hörverarbeitungsort angebracht ist;
einen Hörsignalproduzierer (28, 30), der durch den Rahmen (14) über dem Ohr positioniert ist; und
einen Datenprozessor (12, 41) für eine akustisch evozierte Antwort (AER - Auditory Evoked Response), der betrieblich konfiguriert ist, ein Hörsignal von dem Hörsignalproduzierer (28, 30) zu initiieren und eine Signalverarbeitungsoperation an einem an der Referenz- und Signalelektrode (16, 16') erfassten AER-Signal durchzuführen;
**gekennzeichnet durch**:
einen Datenanalysierer (41, 98) der betrieblich konfiguriert ist, das AER-Signal zu charakterisieren und die Charakteristika mit mindestens einer vorbestimmten AER-Charakteristik zu vergleichen, wobei die mindestens eine vorbestimmte AER-Charakteristik mit einem neurologischen Zustand assoziiert ist.

2. Screening-Vorrichtung nach Anspruch 1, weiterhin umfassend einen freitragenden flexiblen Arm (18), der die Signalelektrode (16) mit dem Rahmen (14) verbindet.

3. Screening-Vorrichtung nach Anspruch 1, weiterhin umfassend eine an dem Rahmen (14) angebrachte zweite Signalelektrode (16").

4. Screening-Vorrichtung nach Anspruch 3, weiterhin umfassend einen Multiplexierungskanal, der von dem AER-Datenprozessor (12, 41) gesteuert wird, um die erste und zweite Signalelektrode (16, 16") selektiv abzutasten.

5. Screening-Vorrichtung nach Anspruch 3, wobei der AER-Datenprozessor (12, 41) weiterhin operativ konfiguriert ist, die erste Signalelektrode (16) mit einer niedrigen Frequenzabtastrate abzutasten und die zweite Signalelektrode (16") mit einer hohen Frequenz abzutasten.

6. Screening-Vorrichtung nach Anspruch 5, weiterhin umfassend einen Multiplexierungskanal, der von dem AER-Datenprozessor (12, 41) gesteuert wird, um die erste und zweite Signalelektrode (16, 16") selektiv abzutasten.

7. Screening-Vorrichtung nach Anspruch 3, weiterhin umfassend einen flexiblen gedruckten Schaltungskabelbaum (50), der die Elektroden (16, 16', 16") und Kommunikationspfade zu dem AER-Datenprozessor (12, 41) enthält und geformt ist um unter dem nachgiebigen Drängen des Rahmens (14) an den Kopf zu passen.

8. Screening-Vorrichtung nach Anspruch 1, weiterhin umfassend eine Testpersonidentifikationseinrichtung (38), wobei der AER-Datenprozessor (12) weiterhin betrieblich konfiguriert ist, eine Testpersonidentifikation mit dem AER-Signal zu assoziieren.

9. Screening-Vorrichtung nach Anspruch 8, wobei die Testpersonidentifikationseinrichtung (38) einen Strichcodescanner umfasst.

10. Screening-Vorrichtung nach Anspruch 8, wobei die Testpersonidentifikationseinrichtung (38) einen Radiofrequenzidentifikationsscanner umfasst.

11. Screening-Vorrichtung nach Anspruch 1, wobei die mindestens eine vorbestimmte AER-Charakteristik eine AER-Legastheniecharakteristik umfasst.

12. Screening-Vorrichtung nach Anspruch 1, weiterhin umfassend eine Kommunikationsbrücke (43), wobei der Datenanalysierer (41, 98) über die Kommunikationsbrücke (43) an den Rahmen (14) gekoppelt ist.

13. Screening-Vorrichtung nach Anspruch 1, wobei der AER-Datenprozessor (12, 41) ein in den Rahmen (14) integriertes Steuermodul (12) umfasst.

14. Screening-Vorrichtung nach Anspruch 1, wobei der Rahmen (14) einen entsorgbaren Abschnitt enthält, der die Elektroden (16, 16', 16") enthält.

15. Screening-Vorrichtung nach Anspruch 1, wobei der AER-Datenprozessor (12, 41) einen digitalen Speicher (74) enthält, der konfiguriert ist, die AER-Daten zu speichern.

16. Screening-Vorrichtung nach Anspruch 1, wobei der AER-Datenprozessor (12, 41) weiterhin betrieblich konfiguriert ist, eine Sequenz von Screening-Tests durchzuführen und mit jedem Test assoziierte AER-Daten in dem digitalen Speicher (74) zu speichern.

17. Screening-Vorrichtung nach Anspruch 16, wobei der digitale Speicher (74) weiterhin ein vorbestimmtes Testprotokoll enthält.

18. Screening-Vorrichtung nach Anspruch 1, wobei der AER-Datenprozessor (12, 41) weiterhin betrieblich konfiguriert ist, eine Benutzerangabe einer Testbedingung zu generieren.

19. Screening-Vorrichtung nach Anspruch 1, wobei der Rahmen betrieblich geformt ist, um zwischen den Ohren über einen Vorderabschnitt eines Patientenkopfs zu verbinden.

20. Screening-Vorrichtung nach Anspruch 1, wobei der Rahmen (14) einen zurückgebogenen Rahmen (14) und ein Paar von an jedem Ende davon angebrachten Ohrmuscheln (26) umfasst.

21. Screening-Vorrichtung nach Anspruch 1, wobei der Rahmen (14) eine Ohrmuschel (26) mit einem daran nach innen befestigten nachgiebigen Abschnitt umfasst.

22. Screening-Vorrichtung nach Anspruch 1, wobei der Rahmen (14) weiterhin eine Ohrmuschel (26) mit einer Elektrode (16") umfasst, die caudad an der Sylvischen Fissur einer Person registriert ist.

23. Verfahren zum Ausführen einer akustisch evozierten Antwort (AER), das Folgendes umfasst:
Positionieren einer Vorrichtung (10) auf dem Kopf einer Person, wobei die Vorrichtung einen Tonproduzierer (28, 30), eine Referenzelektrode (16') und eine Signalelektrode (16) positioniert;
Generieren eines Hörstimulus;
Aufzeichnen von AER-Daten an der Referenz- und Signalelektrode;
**gekennzeichnet durch**:
Verbinden der Vorrichtung mit einem Datenanalysierer (41, 98);
Kennzeichnen der AER-Daten mit dem Datenanalysierer (41, 98) und
Vergleichen der AER-Datencharakteristika mit mindestens einer vorbestimmten AER-Charakteristik mit dem Datenanalysierer (41, 98), wobei die mindestens eine vorbestimmte AER-Charakteristik mit einem neurologischen Zustand assoziiert ist.

24. Verfahren nach Anspruch 23, wobei das Aufzeichnen der AER-Daten weiterhin Folgendes umfasst:
Speichern der AER-Daten auf der Vorrichtung (10) und
Übertragen der gespeicherten AER-Daten an den Datenanalysierer (41, 98).

25. Verfahren nach Anspruch 23, wobei das Positionieren der Vorrichtung (10) auf dem Kopf der Person weiterhin das Positionieren der Person mit dem Gesicht vorwärts oben und das Positionieren der Vorrichtung (10) über einem vorderen Abschnitt des Kopfs der Person umfasst.

26. Verfahren nach Anspruch 23, wobei das Generieren des Hörstimulus weiterhin Folgendes umfasst:
als Reaktion auf das Bestimmen, dass die erfasste Elektrodenspannung einen Schwellwert übersteigt, Auferlegen einer Abtastverzögerung beim Verfolgen eines ruhenden Gehirnzustands.

27. Verfahren nach Anspruch 23, wobei das Generieren des Hörstimulus weiterhin Folgendes umfasst:
Detektieren einer ruhenden Gehirnwelle und
Initiieren eines Hörstimulus bei einer vorbestimmten Steigung der ruhenden Gehirnwelle.

28. Verfahren nach Anspruch 23, weiterhin umfassend:
als Reaktion auf das Bestimmen, dass die AER-Daten ein Artefakt enthalten, Auferlegen einer Abtastverzögerung und Wiederholen einer Hörstimulusepoche und Abtasten von AER-Daten.

29. Verfahren nach Anspruch 23, weiterhin umfassend:
Zugreifen auf ein entfernt gespeichertes Hörtestprotokoll in der Vorrichtung (10) und
Trennen der Vorrichtung (10) vor dem Positionieren einer Vorrichtung (10) auf dem Kopf der Person.

30. Verfahren nach Anspruch 23, wobei die Einrichtung (10) die Referenzelektrode (16'), eine Niederfrequenzsignalelektrode (16") und eine Hochfrequenzsignalelektrode (16) positioniert, wobei das Verfahren weiterhin das Abtasten der Niederfrequenzsignalelektrode (16") mit einer ersten Abtastrate und das Abtasten der Hochfrequenzsignalelektrode (16) mit einer höheren zweiten Abtastrate umfasst.

31. Verfahren nach Anspruch 23, wobei die Einrichtung (10) die Referenzelektrode (16'), eine erste Signalelektrode (16) und eine zweite Signalelektrode (16") positioniert und wobei das Abtasten der ersten und zweiten Frequenzsignalelektrode (16, 16") für jede Elektrode (16, 16") weiterhin Folgendes umfasst:
Erfassen einer EEG-Spannung;
Umwandeln der erfassten Spannung in einen digitalen Wert;
Abtasten des digitalen Werts mit einer vorbestimmten Abtastrate über einen Multiplexierkanal und
Aufzeichnen der multiplexierten digitalen Daten.

32. Verfahren nach Anspruch 31, wobei das Abtasten des digitalen Werts mit einer vorbestimmten Abtastrate über den Multiplexierkanal weiterhin das Abtasten der ersten Signalelektrode (16) mit niedriger Frequenz und Abtasten der zweiten Signalelektrode (16") mit einer hohen Frequenz umfasst.

33. Screening-Vorrichtung nach Anspruch 1, wobei die mindestens eine vorbestimmte AER-Charakteristik mindestens eine Charakteristik enthält ausgewählt aus der Gruppe bestehend aus: einer AER-Legastheniecharakteristik, einer AER-Autismuscharakteristik, einer AER-Schizophreniecharakteristik und einer AER-Aufmerksamkeitsdefizitcharakteristik.

34. Verfahren nach Anspruch 23, wobei die mindestens eine vorbestimmte AER-Charakteristik mindestens eine Charakteristik enthält ausgewählt aus der Gruppe bestehend aus: einer AER-Legastheniecharakteristik, einer AER-Autismuscharakteristik, einer AER-Schizophreniecharakteristik und einer AER-Aufmerksamkeitsdefizitcharakteristik.

## Revendications

1. Dispositif de détection, comprenant :
un cadre (14) formé de façon à pouvoir être en prise avec une tête entre un site de référence, au moins une oreille et un site de détection de signal ;
une électrode de référence (16') fixée au cadre au niveau du site de référence ; une électrode de signal (16) fixée au cadre sur le site de traitement auditif ; un dispositif producteur de signal auditif (28, 30) placé par le cadre (14) sur l'oreille ; et
un processeur de données de réponse évoquée auditive (REA) (12, 41) configuré fonctionnellement pour initier un signal auditif à partir du dispositif producteur de signal auditif (28, 30) et pour réaliser une opération de traitement de signal sur un signal de REA détecté sur les électrodes de référence et de signal (16, 16') ;
**caractérisé par**
un analyseur de données (41, 98) configuré fonctionnellement pour caractériser le signal de REA et pour comparer les caractéristiques à au moins une caractéristique de REA prédéterminée, dans lequel au moins une caractéristique de REA prédéterminée est associée à un état neurologique.

2. Dispositif de détection selon la revendication 1, comprenant en outre un bras flexible en porte à faux (18) raccordant l'électrode de signal (16) au cadre (14).

3. Dispositif de détection selon la revendication 1, comprenant en outre une deuxième électrode de signal (16") fixée au cadre (14).

4. Dispositif de détection selon la revendication 3, comprenant en outre un canal de multiplexage commandé par le processeur de données de REA (12, 41) pour échantillonner sélectivement les première et deuxième électrodes de signal (16, 16").

5. Dispositif de détection selon la revendication 3, dans lequel le processeur de données de REA (12, 41) est en outre configuré fonctionnellement pour échantillonner la première électrode de signal (16) à un taux d'échantillonnage à basse fréquence et pour échantillonner la deuxième électrode de signal (16") à haute fréquence.

6. Dispositif de détection selon la revendication 5, comprenant en outre un canal de multiplexage commandé par le processeur de données de REA (12, 41) pour échantillonner sélectivement les première et deuxième électrodes de signal (16, 16").

7. Dispositif de détection selon la revendication 3, comprenant en outre un harnais (50) de circuit imprimé flexible contenant les électrodes (16, 16', 16") et des voies de communication jusqu'au processeur de données de REA (12, 41) et conçu pour se conformer à la tête sous la poussée élastique du cadre (14).

8. Dispositif de détection selon la revendication 1, comprenant en outre un dispositif d'identification d'un sujet de test (38), le processeur de données de REA (12) étant en outre configuré fonctionnellement pour associer une identification d'un sujet de test au signal de REA.

9. Dispositif de détection selon la revendication 8, dans lequel le dispositif d'identification du sujet de test (38) comprend un scanner de code-barres.

10. Dispositif de détection selon la revendication 8, dans lequel le dispositif d'identification du sujet de test (38) comprend un scanner d'identification d'une radiofréquence.

11. Dispositif de détection selon la revendication 1, dans lequel au moins une caractéristique de REA prédéterminée comprend une caractéristique de REA de la dyslexie.

12. Dispositif de détection selon la revendication 1, comprenant en outre un lien de communication (43) dans lequel l'analyseur de données (41, 98) est couplé au cadre (14) via le lien de communication (43).

13. Dispositif de détection selon la revendication 1, dans lequel le processeur de données de REA (12, 41) comprend un module de contrôle (12) qui fait partie intégrante du cadre (14).

14. Dispositif de détection selon la revendication 1, dans lequel le cadre (14) comprend une partie jetable qui comprend les électrodes (16, 16', 16").

15. Dispositif de détection selon la revendication 1, dans lequel le processeur de données de REA (12, 41) comprend une mémoire numérique (74) configurée pour stocker les données de REA.

16. Dispositif de détection selon la revendication 1, dans lequel le processeur de données de REA (12, 41) est en outre configuré fonctionnellement de façon à réaliser une séquence de tests de détection et à stocker dans la mémoire numérique (74) des données de REA associées à chaque test.

17. Dispositif de détection selon la revendication 16, dans lequel la mémoire numérique (74) comprend en outre un protocole de test prédéterminé.

18. Dispositif de détection selon la revendication 1, dans lequel le processeur de données de REA (12, 41) est en outre configuré fonctionnellement pour générer une indication d'utilisateur d'une condition de test.

19. Dispositif de détection selon la revendication 1, dans lequel le cadre est conçu fonctionnellement pour être raccordé entre les oreilles sur une partie frontale de la tête d'un patient.

20. Dispositif de détection selon la revendication 1, dans lequel le cadre (14) comprend un cadre recourbé (14) et une paire d'écouteurs (26) fixés à chaque extrémité de celui-ci.

21. Dispositif de détection selon la revendication 1, dans lequel le cadre (14) comprend un écouteur (26) ayant une partie élastique fixée vers l'intérieur de celle-ci.

22. Dispositif de détection selon la revendication 1, dans lequel le cadre (14) comprend en outre un écouteur (26) ayant une électrode (16") enregistrée au niveau caudal par rapport à la fissure sylvienne d'un sujet.

23. Procédé de réalisation d'une réponse évoquée auditive (REA) comprenant :
le placement d'un dispositif (10) sur la tête d'un sujet, le dispositif mettant en place un dispositif producteur de son (28, 30), une électrode de référence (16') et une électrode de signal (16) ;
la génération d'un stimulus auditif ;
l'enregistrement des données de REA sur les électrodes de référence et les électrodes de signal ;
**caractérisé par**
le raccordement du dispositif à un analyseur de données (41, 98) ;
la caractérisation des données de REA avec l'analyseur de données (41, 98) ; et
la comparaison des caractéristiques de données de REA à au moins une caractéristique de REA prédéterminée avec l'analyseur de données (41, 98), dans lequel au moins une caractéristique de REA prédéterminée est associée à un état neurologique.

24. Procédé selon la revendication 23, dans lequel les données de REA comprennent en outre :
le stockage des données de REA sur le dispositif (10) ; et
la transmission des données de REA stockées à l'analyseur de données (41, 98).

25. Procédé selon la revendication 23, dans lequel le placement du dispositif (10) sur la tête du sujet comprend en outre le placement du sujet le visage tourné vers l'avant et le placement du dispositif (10) sur une partie avant de la tête du sujet.

26. Procédé selon la revendication 23, dans lequel la génération du stimulus auditif comprend en outre :
en réponse à la détermination de la tension d'électrode détectée dépassant un seuil, l'imposition d'un délai d'échantillonnage dans la poursuite d'un état cérébral au repos.

27. Procédé selon la revendication 23, dans lequel la génération du stimulus auditif comprend en outre :
la détection d'une onde cérébrale au repos ; et
l'initiation du stimulus auditif sur une pente prédéterminée de l'onde cérébrale au repos.

28. Procédé selon la revendication 23, comprenant en outre :
en réponse à la détermination des données de REA susceptibles de contenir un artefact, l'imposition d'un délai d'échantillonnage et la répétition d'une époque de stimulus auditif et l'échantillonnage de données de REA.

29. Procédé selon la revendication 23, comprenant en outre :
l'accession à un protocole de test auditif enregistré à distance dans le dispositif (10) ; et
la déconnexion du dispositif (10) avec le placement d'un dispositif (10) sur la tête du sujet.

30. Procédé selon la revendication 23, dans lequel le dispositif (10) place l'électrode de référence (16'), une électrode de signal à basse fréquence (16") et une électrode de signal à haute fréquence (16), le procédé comprenant en outre l'échantillonnage de l'électrode de signal à basse fréquence (16") au premier taux d'échantillonnage et l'échantillonnage de l'électrode de signal à haute fréquence (16) à un deuxième taux d'échantillonnage supérieur.

31. Procédé selon la revendication 23, dans lequel le dispositif (10) place l'électrode de référence (16'), une première électrode de signal (16) et une deuxième électrode de signal (16") et dans lequel l'échantillonnage des première et deuxième électrodes de signal de fréquence (16, 16") comprend en outre pour chaque électrode (16, 16") :
la détection d'une tension d'EEG ;
la conversion de la tension détectée en une valeur numérique ;
l'échantillonnage de la valeur numérique à un taux d'échantillonnage prédéterminé sur un canal de multiplexage ; et
l'enregistrement des données numériques multiplexées.

32. Procédé selon la revendication 31, dans lequel l'échantillonnage de la valeur numérique à un taux d'échantillonnage prédéterminé sur le canal de multiplexage comprend en outre l'échantillonnage de la première électrode de signal (16) à basse fréquence et l'échantillonnage de la deuxième électrode de signal (16") à haute fréquence.

33. Dispositif de détection selon la revendication 1, dans lequel au moins une caractéristique de REA prédéterminée comprend au moins une caractéristique choisie dans le groupe comprenant une caractéristique de REA de la dyslexie, une caractéristique de REA de l'autisme, une caractéristique de REA de la schizophrénie et une caractéristique de REA du trouble de déficit d'attention.

34. Procédé selon la revendication 23, dans lequel au moins une caractéristique de REA prédéterminée comprend au moins une caractéristique choisie dans le groupe comprenant une caractéristique de REA de la dyslexie, une caractéristique de REA de l'autisme, une caractéristique de REA de la schizophrénie et une caractéristique de REA du trouble de déficit d'attention.
